# EUROPEAN PATENT SPECIFICATION

(11) **EP 4 563 182 B1**
(45) Date of publication and mention of the grant of the patent: **21.10.2026**
(21) Application number: 23897366.3
(22) Date of filing: 01.11.2023
(51) Int. Cl.: A61M 25/10

(54) **BALLOON CATHETER AND METHOD OF MANUFACTURING SAME**
BALLONKATHETER UND VERFAHREN ZUR HERSTELLUNG DAVON
CATHÉTER À BALLONNET ET SON PROCÉDÉ DE FABRICATION

(30) Priority: 30.11.2022 JP 2022192303
(43) Date of publication of application: 04.06.2025
(73) Proprietor: Nipro Vascular Corporation, Nagoya-shi, Aichi 460-0008 (JP)
(72) Inventor: OKAMOTO, Mitsumasa, Seto-shi, Aichi 489-0976 (JP); KONDO, Shoma, Seto-shi, Aichi 489-0976 (JP); NAKAMURA, Yuta, Seto-shi, Aichi 489-0976 (JP); YAMAMOTO, Shuhei, Seto-shi, Aichi 489-0976 (JP)
(74) Representative: Prüfer & Partner mbB Patentanwälte · Rechtsanwälte
(86) International application number: PCT/JP2023/039483
(87) International publication number: WO 2024/116710

(56) References cited:
- WO-A1-2020/012850
- WO-A1-2020/012850
- WO-A1-2020/250611
- WO-A1-2020/255923
- WO-A1-2020/255923
- WO-A1-2022/196166
- JP-A- 2021 104 223
- JP-A- 2021 104 223
- US-A1- 2003 004 535
- US-A1- 2010 036 314
- US-A1- 2020 215 310

## Description

### Cross-Reference to Related Application

The present application is based on Japanese Patent Application No. 2022-192303 filed on November 30, 2022.

### Technical Field

The present disclosure relates to a balloon catheter and a method of producing a balloon catheter.

### Background Art

A balloon catheter has been used in percutaneous transluminal angioplasty (PTA), percutaneous transluminal coronary angioplasty (PTCA) and other surgeries. The balloon catheter includes an inflatable and deflatable balloon at a tip-end portion of the balloon catheter. The balloon may be deflated and inserted into a section of the blood vessel that is narrowed or obstructed by a lesion and then inflated to dilate the section. The balloon includes a straight tube portion and two tapered portions. The straight tube portion has a cylindrical shape with a diameter that is to be maximum when the balloon is inflated. The tapered portions are disposed on a base-end side and a tip-end side of the straight tube portion. Each tapered portion has a diameter that decreases as a distance from the straight tube portion increases.

A balloon catheter may include a linear protrusion that protrudes from a surface of a straight tube portion and extends along the surface of the straight tube portion (see PLT1 for example). When the balloon is inflated in a section with a lesion, the linear protrusions dig into the lesion to create incisions in the lesion. The section with the lesion can be easily dilated with the incisions as a trigger of the dilation.

### Citation List

### Patent Literature

PLT1: International Publication WO2020/255923. Furthermore, balloon catheters or methods of manufacturing the same are known, for example, from JP 2021 104223 A, US 2020/215310 A1, WO 2020/255923 A1, WO 2020/012850 A1, US 2003/004535 A1 and WO 2022/196166 A1.

### Summary of Invention

The aforementioned objectives are achieved according to the invention by means of a balloon catheter according to claim 1 and equally by means of a method of producing such a balloon catheter according to claim 3. Preferred embodiments are defined in the dependent claims.

### Technical Problem

When the balloon is inflated to dilate the section with the lesion in the body, the lesion may push back the balloon and thus balloon may slip in the axial direction. If that happens, the balloon may not be able to properly dilate the section with the lesion.

To reduce the slipping of the balloon during the inflation of the balloon, surface roughness of the surface of the straight tube portion of the balloon may be increased. However, this may increase a sliding resistance of the balloon during the insertion of the balloon into a tube such as a blood vessel and may decrease ease in passing of the balloon through the tube.

The present disclosure has been made in view of the above circumstances. A main objective of the present disclosure is to provide a balloon catheter that includes a balloon that is less likely to slip during inflation of the balloon although ease in passing of the balloon through a tube is less likely to decrease, and to provide a method of producing the balloon catheter.

### Solution to Problem

To solve the problem described above, a first aspect of the present disclosure provides a balloon catheter that includes a balloon and a linear protrusion. The balloon is inflatable and deflatable. The balloon includes a straight tube portion that is in a cylindrical shape with a diameter to be maximum when the balloon is inflated. The linear protrusion protrudes from a surface of the straight tube portion and extends along the surface of the straight tube portion. The linear protrusion has a surface that includes at least one rough surface region with surface roughness greater than surface roughness of the surface of the straight tube portion.

In the first aspect, the linear protrusion is disposed on the straight tube portion of the balloon and the rough surface region that has the surface roughness greater than the surface roughness of the surface of the straight tube portion is formed in the surface of the linear protrusion. According to the configuration, when the balloon is inflated and the linear protrusion is pressed against a subject in a target section to be dilated such as a section with a lesion, the rough surface region of the linear protrusion contacts the subject in the target section to be dilated. Therefore, the balloon is less likely to slip during inflation of the balloon.

The surface roughness of the surface of the straight tube portion is less than the surface roughness of the rough surface region of the linear protrusion. Because an area of the surface of the straight tube portion is significantly larger than an area of the surface of the linear protrusion, a sliding resistance of the balloon during insertion of the balloon into a tube such as a blood vessel can be reduced. According to the configuration, the balloon is less likely to slip during the inflation of the balloon while the ease in passing of the balloon through the tube is less likely to decrease.

In a second aspect of the present disclosure, the balloon of the balloon catheter in the first aspect includes a wing that is formed when the balloon is deflated and folded along an outer periphery of the balloon. Further, the linear protrusion extends in an axial direction of the balloon. The linear protrusion is covered by the wing from an outer side when the balloon is deflated.

According to the second aspect, because the linear protrusion is covered by the wing from the outer side when the balloon is deflated, the at least one rough surface region of the linear protrusion is less likely to slide on a tube wall during insertion of the deflated balloon into the tube. Therefore, the ease in passing of the balloon is further less likely to decrease.

In a third aspect of the present disclosure, the at least one rough surface region of the balloon of the balloon catheter in the second aspect is formed only in an intermediate portion of the linear protrusion with respect to a longitudinal direction of the linear protrusion, or the surface roughness of the at least one rough surface region decreases toward an end of the linear protrusion with respect to the longitudinal direction.

In the second aspect described earlier, the linear protrusion may not be entirely covered by the wing. For example, an end portion of the linear protrusion may stick out of the wing. The third aspect is conceived in view of such circumstances. The third aspect provides the configuration includes the at least one rough surface region that is formed only in the intermediate portion of the linear protrusion, or the configuration that includes the at least one rough surface region with the surface roughness that decreases toward the end of the linear protrusion with respect to the longitudinal direction.

According to the former configuration, because the at least one rough surface region is not formed in the end portion of the linear protrusion with respect to the longitudinal direction, the sliding resistance is less likely to increase even if the end portion of the linear protrusion sticks out of the wing and slides on the tube wall during the insertion of the balloon that is deflated into a tube. Therefore, the ease in passing of the balloon is further less likely to decrease. According to the latter configuration, because the surface roughness of the at least one rough surface region in the end portion is less, similar effects as the effects achieved by the former configuration can be achieved.

In a fourth aspect of the present disclosure, the at least one rough surface region of the balloon catheter in the first aspect or the second aspect includes rough surface regions in the surface of the linear protrusion. The rough surface regions are arranged such that a density of the rough surface regions increases toward a peak of the linear protrusion.

When the balloon is inflated until the linear protrusion is pressed against a subject in a target section to be dilated such as a lesion, the peak of the linear protrusion mainly contacts the subject. In the fourth aspect, the rough surface regions in the surface of the linear protrusion are arranged such that the density of the rough surface regions increases toward the peak of the linear protrusion. According to the configuration, the slipping of the balloon during the inflation of the balloon is properly reduced. Because the rough surface regions are arranged such that the density of the rough surface regions decreases toward a base of the linear protrusion (opposite from the peak), the sliding resistance of the balloon during the insertion of the balloon into the tube is reduced in comparison to a configuration in which the rough surface regions are arranged such that the density of the rough surface regions is high for the entire area of the surface of the linear protrusion. Therefore, the slipping of the balloon is properly reduced although the ease in passing of the balloon is further less likely to decrease.

In a fifth aspect of the present disclosure, the surface roughness of the at least one rough surface region of the balloon catheter in the first aspect or the second aspect increases toward the peak of the linear protrusion.

According to the fifth aspect, because the surface roughness of the rough surface region increases toward the peak of the linear protrusion, the slipping of the balloon during the inflation of the balloon is properly reduced. Because the surface roughness of the rough surface region decreases toward the base of the protrusion, the sliding resistance of the balloon during the insertion of the balloon into the tube is reduced in comparison to the configuration in which the surface roughness is greater for the entire area of the surface of the linear protrusion. According to the configuration, the slipping of the balloon is properly reduced although the ease in passing of the balloon is further less likely to decrease, similar to the fourth aspect.

In a sixth aspect of the present disclosure, the balloon in the first aspect or the second aspect includes two tapered portions that sandwich the straight tube portion. The tapered portions decrease in diameter toward farther sides from the straight tube portion. The tapered portions include surfaces with surface roughness greater than the surface roughness of the surface of the straight tube portion. The surface roughness of the surfaces of the tapered portions is less than the surface roughness of the at least one rough surface region.

During dilation of the section with the lesion using the tapered portions of the balloon, the tapered portions contact the lesion at an angle and thus the lesion may push back the tapered portions, which may cause slipping of the balloon in the axial direction. In the sixth aspect, the surface roughness of the surfaces of the tapered portions is greater than the surface roughness of the surface of the straight tube portion. According to the configuration, the surfaces of the tapered portion contact the lesion, which reduces the slipping of the tapered portions in the axial direction and the slipping of the balloon in the axial direction. Because the surface roughness of the surfaces of the tapered portions is less than the surface roughness of the rough surface region of the linear protrusion, the effects described above can be achieved without a reduction in ease in passing of the balloon into the tube.

A seventh aspect of the present disclosure provides a method of producing the balloon catheter. A parison in a tubular shape is a base material of the balloon. The parison includes a protrusion on an outer periphery of the parison. The parison is to be formed into the linear protrusion. A mold that is used for producing the balloon includes an internal space for forming the balloon. The internal space includes a space section for forming the straight tube portion. The mold includes an inner wall surface that defines the internal space and includes a wall surface section that defines the space section. The mold includes a groove in the wall surface section for forming the linear protrusion. The mold includes an inner surface that defines the groove and includes a rough surface section with surface roughness greater than surface roughness of the wall surface section. The method includes an expanding process of expanding the parison in the internal space until the outer periphery of the parison is pressed against the inner wall surface and the parison is pushed into the groove to form the linear protrusion. The at least one rough surface region is formed in the surface of the linear protrusion by pushing the protrusion into the groove and pressing the protrusion against the rough surface section in the expanding process.

In the seventh aspect, the parison is expanded in the internal space of the mold until the protrusion of the parison is pushed into the groove in the wall surface section of the mold and the linear protrusion is formed. By pushing the protrusion of the parison into the groove and pressing the protrusion against the rough surface section, the rough surface region is formed in the surface of the linear protrusion. Because the rough surface region is formed during the expansion of the parison, a downstream process to form the rough surface region, such as scraping of the surface of the linear protrusion after the balloon with the linear protrusion is formed through the expansion of the parison, is not required. According to the seventh aspect, the balloon catheter in the first aspect is relatively easily produced.

In an eighth aspect of the present disclosure, the expanding process in the method of producing the balloon catheter in the seventh aspect includes a first expanding step and the second expanding step. The first expanding step includes pressurizing an inside of the parison at a first pressure to expand the parison until the protrusion is inserted into the groove. The second expanding step includes pressurizing the inside of the parison at a second pressure that is higher than the first pressure to push the protrusion into the groove until the protrusion is pressed against the rough surface section.

In the eighth aspect, the inside of the parison is pressurized at the first pressure that is relatively lower to expand the parison until the protrusion is inserted into the groove (the first expanding step). Through the step, the parison is relatively slowly expanded and thus the protrusion of the parison is properly inserted into the groove. Then, the inside of the parison is pressurized at the second pressure that is higher than the first pressure to push the protrusion into the groove until the protrusion is pressed against the rough surface section (the second expanding step). Through the step, the protrusion is pushed into the groove and thus the protrusion is pressed against the rough surface section. Therefore, the rough surface region is properly formed in the surface of the linear protrusion.

In a ninth aspect of the present disclosure, the first expanding step in the method of producing the balloon catheter in the eighth aspect includes heating the parison at a first temperature and the second expanding step includes heating the parison at a second temperature that is higher than the first temperature.

In the ninth aspect, the parison is heated in the second expanding step at the temperature that is higher than the temperature in the first expanding step (the second temperature). That is, the protrusion of the parison that is softened is pushed into the groove. Therefore, the protrusion is more easily pressed against the rough surface section and thus the rough surface region is properly formed in the linear protrusion.

In a tenth aspect of the present disclosure, in the method of producing the balloon catheter in any one of the seventh to the ninth aspects, the protrusion has a height greater than a depth of the groove.

In the tenth aspect, because the height of the protrusion of the parison is greater than the depth of the groove, the protrusion is properly inserted into the groove until the protrusion reaches a bottom of the groove and thus the protrusion is properly pressed against the rough surface section. Therefore, the rough surface region is properly formed even in an area adjacent to the peak of the linear protrusion. With such a rough surface region, the slipping of the balloon during the inflation of the balloon is properly reduced.

In an eleventh aspect of the present disclosure, in the method of producing the balloon catheter in any one of the seventh to the ninth aspects, the groove has a cross section in a V-shape and the protrusion has a cross section in an inverted V-shape. The inner surface that defines the groove includes two side surfaces that form the V-shape. The protrusion includes a surface that includes two side surfaces that form the inverted V-shape. The side surfaces of the protrusion form an angle that is greater than an angle formed by the side surfaces that define the groove.

In the eleventh aspect, the cross section of the protrusion of the parison is in the inverted V-shape and the cross section of the groove is in the V-shape. The angle formed by the side surfaces of the protrusion is greater than the angle formed by the side surfaces that define the groove. According to the configuration, the protrusion deforms along the side surfaces that define the groove during the pushing of the protrusion into the groove and thus the protrusion is pressed against the side surfaces that define the groove (or rather the rough surface section). That is, the configuration described above is preferable for the formation of the rough surface region in the linear protrusion in the inverted V-shape.

In a twelfth aspect of the present disclosure, in the method of producing the balloon catheter in the eleventh aspect, an angle of each of the side surfaces relative to a width direction of the groove is greater at an opening section of the groove in comparison to a bottom section of the groove.

In the eleventh aspect, the cross section of the groove is in the V-shape and the cross section of the protrusion is in the inverted V-shape. Therefore, the protrusion may slide on the side surfaces that define the groove after the protrusion is inserted into the groove, which may cause a problem such as improper formation of the rough surface region in the side surfaces of the linear protrusion. The twelfth aspect is conceived in view of such circumstances. The angle of each of the side surfaces that define the groove relative to the width direction of the groove is greater at the opening section of the groove in comparison to the bottom section of the groove. According to the configuration, the protrusion inserted into the groove is less likely to slide along the side surfaces that define the groove. Therefore, the problem due to the sliding of the protrusion is less likely to occur.

In a thirteenth aspect of the present disclosure, in the method of producing the balloon catheter in any one of the seventh to the ninth aspects, the groove has a width that decreases toward a bottom of the groove. The inner surface that defines the groove includes two side surfaces adjacent to each other and joined to each other at a bottom of the groove. The expanding process includes pushing the protrusion into the groove until the protrusion contacts the side surfaces that define the groove and then the bottom of the groove.

According to the thirteenth aspect, the protrusion is pushed to the bottom of the groove while the protrusion is being deformed along the side surfaces that define the groove during the pushing of the protrusion into the groove. Therefore, the protrusion is properly pressed against the entire area of the side surfaces that define the groove (or rather the rough surface section) and thus the rough surface region is properly formed in the linear protrusion.

### Brief Description of Drawings

The above-described object and any other object, features, and advantages of the present disclosure will be further clarified by the following detailed description made with reference to the attached drawings.
[FIG. 1] A schematic overall side view illustrating a configuration of a balloon catheter.
[FIG. 2] (a) A side view illustrating a configuration of a balloon that is inflated and therearound, (b) a cross-sectional view cut along line A-A in FIG. 2(a), (c) a magnified cross-sectional view illustrating a linear protrusion that is illustrated in FIG. 2(b).
[FIG. 3] A side view of the balloon that is inflated and therearound with a lengthwise cross-sectional view of the balloon and an outer tube.
[FIG. 4] (a) A side view of the balloon that is deflated and therearound, (b) a cross-sectional view cut along line B-B in FIG. 4(a).
[FIG. 5] (a) A cross-sectional view illustrating a configuration of a mold, (b) a cross-sectional view cut along line C-C in FIG. 5(a), (c) a magnified cross-sectional view illustrating a groove that is illustrated in FIG. 5(b).
[FIG. 6] A perspective view of a parison.
[FIG. 7] (a) A cross-sectional view illustrating the parison that is expanded in an internal space of the mold, (b) a cross-sectional view cut along line D-D in FIG. 7(a).
[FIG. 8] A cross-sectional view illustrating steps through which a protrusion of the parison is pushed into the groove of the mold in an expanding process.
[FIG. 9] A side view illustrating a configuration of a balloon and therearound in another embodiment.
[FIG. 10] A magnified side view of a linear protrusion with rough surface regions in another embodiment.
[FIG. 11] A cross-sectional view of a groove in a mold in another embodiment.
[FIG. 12] A side view illustrating a configuration of a balloon and therearound in another embodiment.

### Description of Embodiments

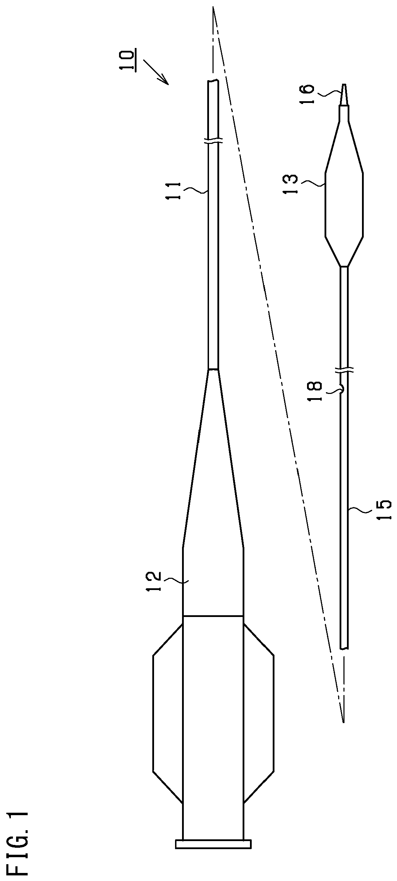

An embodiment of the present disclosure will be described with reference to the drawings. FIG. 1 is a schematic overall side view illustrating a configuration of a balloon catheter.

As illustrated in FIG. 1, a balloon catheter 10 includes a catheter tube 11, the hub 12, and a balloon 13. The hub 12 is connected to a base-end portion (a proximal portion) of the catheter tube 11. The balloon 13 is mounted to a tip-end portion (a distal portion) of the catheter tube 11.

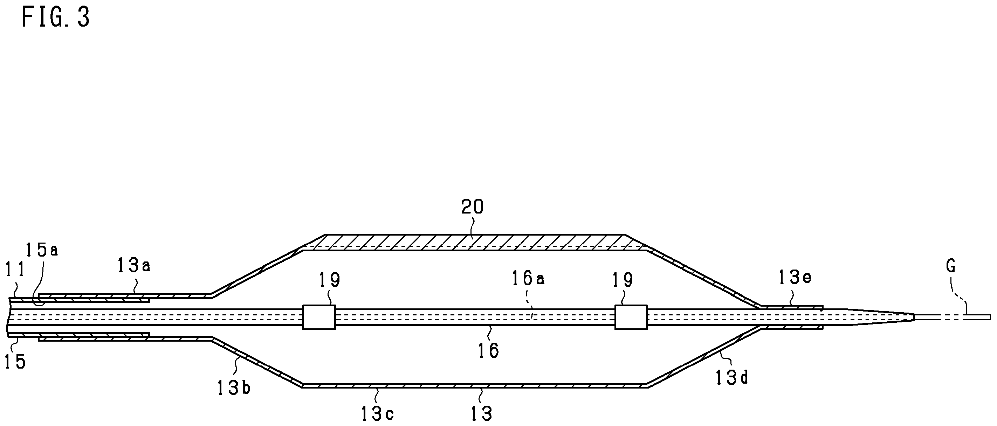

The catheter tube 11 includes an outer tube 15 and an inner tube 16 that is inserted in the outer tube 15. The outer tube 15 is made of a resin material and formed in a tubular shape. The outer tube 15 includes a fluid lumen 15a inside (see FIG. 3). The fluid lumen 15a extends through the outer tube 15 in an axial direction of the outer tube 15 for an entire length. A base-end portion of the outer tube 15 is joined to the hub 12, and the tip-end portion of the outer tube 15 is joined to the balloon 13. The fluid lumen 15a of the outer tube 15 is communicated with an internal space of the hub 12 and an internal space of the balloon 13. For inflation and deflation of the balloon 13, a compressed fluid is passed through the fluid lumen 15a of the outer tube 15.

The outer tube 15 may be composed of multiple tubes that are disposed in a row with respect to the axial direction and joined to one another. One of the multiple tubes on the base-end side may be made of a metal material and another one of the multiple tubes on the tip-end side may be made of a resin material.

The inner tube 16 is made of a resin material and formed in a tubular shape. The inner tube 16 includes a guidewire lumen 16a inside (see FIG. 3). A base-end portion of the inner tube 16 is joined to a middle portion of the outer tube 15 with respect to the axial direction of the outer tube 15. A portion of the inner tube 16 on the tip-end side extends farther than a tip of the outer tube 15 and inserted in an internal space of the balloon 13. A tip-end portion of the inner tube 16 is joined to a tip-end portion of the balloon 13.

The guidewire lumen 16a of the inner tube 16 is provided for passing a guidewire G. A base-end opening of the guidewire lumen 16a is located in the middle of the balloon catheter 10 with respect to the axial direction of the balloon catheter 10. That is, the balloon catheter 10 is classified as an RX-type catheter. A base-end opening 18 of the guidewire lumen 16a may be located at a base-end portion of the balloon catheter 10. This type of the balloon catheter 10 may be classified as an over-the-wire-type catheter.

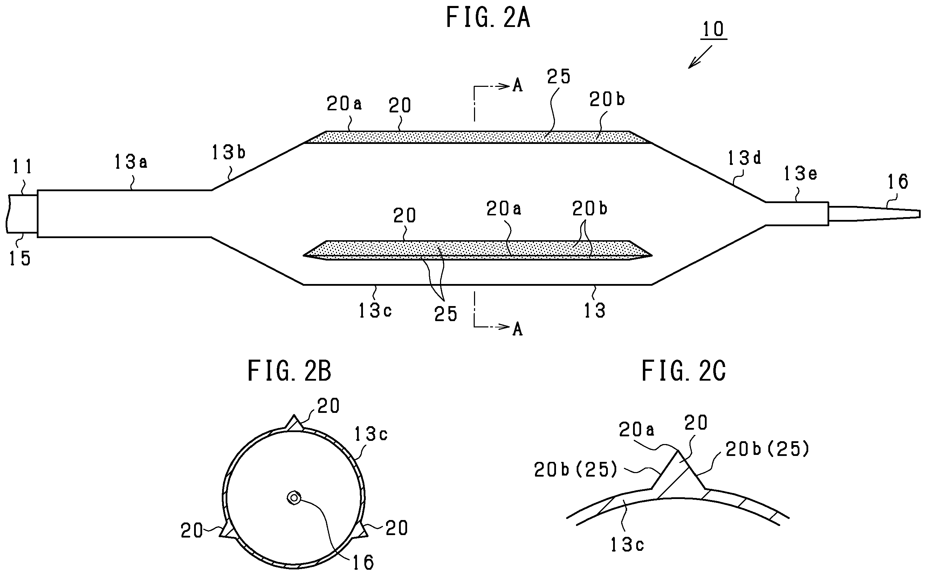

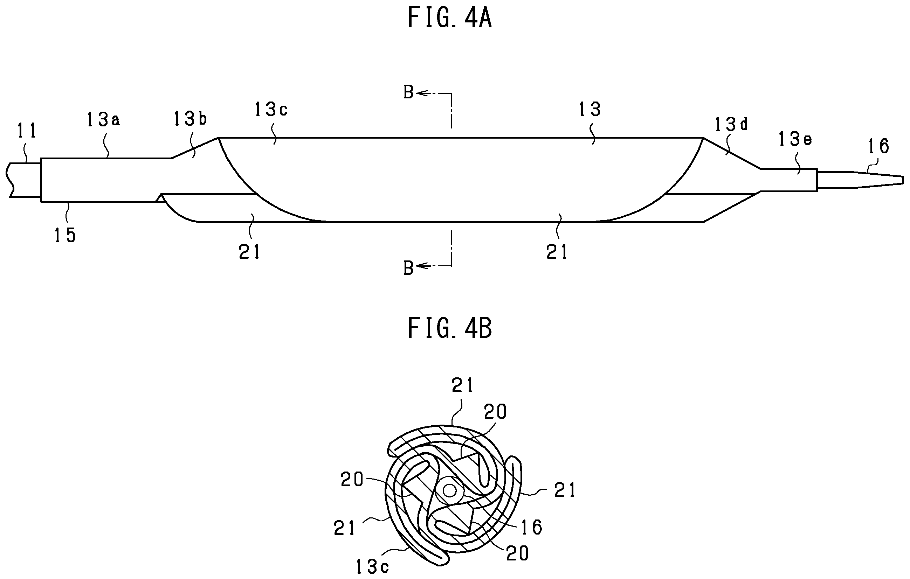

A configuration of the balloon 13 and therearound will be described with reference to FIGS. 2(a) to 4(b). FIG. 2(a) is a side view illustrating the configuration of the balloon 13 that is inflated and therearound. FIG. 2(b) is a cross-sectional view cut along line A-A in FIG. 2(a). FIG. 2(c) is a magnified cross-sectional view of the linear protrusion 20 that is illustrated in FIG. 2(b). FIG. 3 is a side view illustrating a configuration of the balloon 13 that is inflated and therearound with a lengthwise cross-sectional view of the balloon 13 and the outer tube 15. FIG. 4(a) is a side view illustrating a configuration of the deflated balloon 13 and therearound. FIG. 4(b) is a cross-sectional view cut along line B-B in FIG. 4(a).

The balloon 13 is made of a thermoplastic resin material such as polyamide elastomer. As illustrated in FIG. 2(a) and FIG. 3, the balloon 13 is formed in a cylindrical shape (a tubular shape) with a circular cross section as a whole. Specifically, the balloon 13 includes a base-end leg portion 13a, a base-end tapered portion 13b, a straight tube portion 13c, a tip-end tapered portion 13d, and a tip-end leg portion 13e in this sequence from the base-end side toward the tip-end side.

The base-end leg portion 13a is joined to the tip-end portion of the outer tube 15. The base-end tapered portion 13b increases in diameter from the tip-end of the base-end leg portion 13a toward the tip-end side. That is, the base-end tapered portion 13b is in a tapered shape. The straight tube portion 13c extends from the tip-end side of the base-end tapered portion 13b toward the tip-end side with a constant diameter. That is, the straight tube portion 13c is in a round tubular shape (a cylindrical shape). The straight tube portion 13c has a diameter that is to be maximum when the balloon 13 is inflated. The tip-end tapered portion 13d extends from the tip-end of the straight tube portion 13c toward the tip-end side with a diameter that decreases toward the tip-end side. That is, the tip-end tapered portion 13d is in a tapered shape. The tip-end leg portion 13e is joined to the tip-end portion of the inner tube 16.

When the compressed fluid is supplied to the internal space of the balloon 13 via the fluid lumen 15a of the outer tube 15, the balloon 13 inflates. When the negative pressure is applied to the fluid lumen 15a of the outer tube 15 and the compressed fluid is discharged from the internal space of the balloon 13, the balloon 13 deflates. As illustrated in FIGS. 4(a) and 4(b), the balloon 13 that is deflated includes multiple (three in this embodiment) wings 21 that appear when the balloon 13 is deflated. The wings 21 are disposed at predetermined intervals (specifically, equal intervals) in the circumferential direction of the balloon 13. Each of the wings 21 extends in the axial direction over the tapered portions 13b, 13d and the straight tube portion 13c. When the balloon 13 is deflated, the wings 21 are folded along the outer periphery of the balloon 13 and wound around the inner tube 16.

Two contrast rings 19 are attached to the inner tube 16 inside the balloon 13. The contrast rings 19 are for improving visibility of the balloon 13 in x-ray images to easily position the balloon 13 to a target section for treatment.

In the balloon catheter 10, linear protrusions 20 protrude from an outer surface of the straight tube portion 13c of the balloon 13 and linearly extend along the outer surface. The linear protrusions 20 are for creating incisions in a lesion during dilation of a section with the lesion by inflating the balloon 13. With the balloon catheter 10, the section with the lesion can be easily dilated by creating incisions in the lesion with the linear protrusions 20 and using the incisions as a trigger of the dilation. That is, the balloon catheter 10 is a balloon catheter having a scoring function.

The linear protrusions 20 protrude from the outer surface of the straight tube portion 13c and extend in the axial direction of the balloon 13 along the outer surface of the straight tube portion 13c. Specifically, the linear protrusions 20 extend in the axial direction for the entire length of the straight tube portion 13c. The linear protrusions 20 are disposed at predetermined intervals (specifically, equal intervals) in the circumferential direction of the balloon 13. In this embodiment, three linear protrusions 20 are disposed. The linear protrusions 20 are integrally formed with the balloon 13.

Each linear protrusion 20 has a widthwise cross section (specifically, a cross section along a direction perpendicular to the longitudinal direction of the linear protrusion 20) in an inverted V-shape that protrudes outward in the radial direction of the balloon 13, specifically, in a triangular shape. A protruding end of the linear protrusion 20 is a peak 20a. The linear protrusion 20 includes two side surfaces 20b that are adjacent to each other and joined to each other at the peak 20a.

The side surfaces 20b of the linear protrusion 20 includes rough surface regions 25 that have surface roughness greater than surface roughness of the surface of the straight tube portion 13c. The linear protrusions 20 include the rough surface regions 25, respectively. In FIG. 2(a), the rough surface regions 25 are indicated with dot-hatching patterns. The rough surface regions 25 are present for the entire areas of the side surfaces 20b of the linear protrusion 20 (i.e., the entire surfaces of the linear protrusion 20). The surface roughness of the rough surface regions 25 is constant for the entire areas of the rough surface regions 25. The surface roughness of the rough surface regions 25 of all the linear protrusions 20 are equal. The surface roughness of the surface of the balloon 13 is constant for the entire area of the surface of the balloon 13.

In this disclosure, the expression "surface roughness" means calculated mean roughness Ra defined in the Japanese industrial standards (JIS) B0601:2001. The calculated mean roughness Ra is measured according to JIS B0601:2001 using instrument specified in JIS B0651:2001.

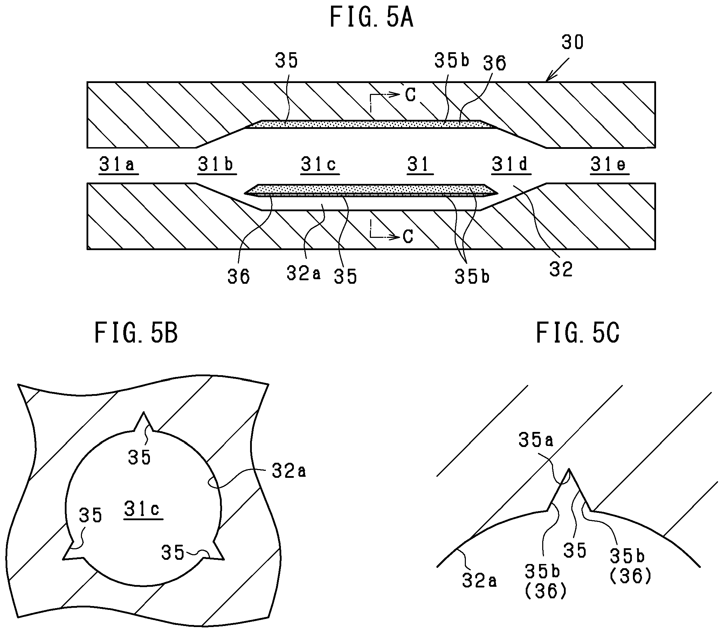

A method of producing the balloon 13 that is described above will be described. The balloon 13 is produced using a mold 30. First, a configuration of the mold 30 will be described with reference to FIGS. 5(a) to 5(c). FIG. 5(a) is a cross-sectional view illustrating the configuration of the mold 30. FIG. 5(b) is a cross-sectional view cut along line C-C in FIG. 5(a). FIG. 5(c) is a magnified cross-sectional view of a groove 35 illustrated in FIG. 5(b).

As illustrated in FIG. 5(a), the mold 30 in a rectangular shape includes an internal space 31 in which the balloon 13 is formed. The internal space 31 is in an elongated shape that extends in the longitudinal direction of the mold 30 and corresponds to the shape of the balloon 13. As illustrated in FIG. 5(b), the internal space 31 has a cross section (a cross section along a direction perpendicular to the longitudinal direction of the internal space 31) in a circular shape for an entire length of the internal space 31 in the longitudinal direction.

The internal space 31 includes a space section 31a for forming the base-end leg portion 13a of the balloon 13, a space section 31b for forming the base-end tapered portion 13b, a space section 31c for forming the straight tube portion 13c, a space section 31d for forming the tip-end tapered portion 13d, and space section 31e for forming the tip-end leg portion 13e. The space sections 31a to 31e are in shapes corresponding to the portions 13a to 13e of the balloon 13, respectively.

The mold 30 includes an inner wall surface 32 that defines the internal space 31. The inner wall surface 32 surrounds the internal space 31. The surface roughness of the inner wall surface 32 is constant for the entire area of the inner wall surface 32. Multiple (specifically, three) grooves 35 are formed in an inner wall surface section 32a of the inner wall surface 32 (corresponding to a wall surface section). The inner wall surface section 32a defines the space section 31c of the internal space 31 for the straight tube portion 13c (corresponding to a space section recited in the claims). The grooves 35 extend in the longitudinal direction of the internal space 31. The grooves 35 are at equal intervals in the circumferential direction of the inner wall surface 32.

As illustrated in FIG. 5(c), each groove 35 has a widthwise cross section in a valley shape, that is, a V-shape along a direction perpendicular to the longitudinal direction of the groove 35. The cross section of the groove 35 corresponds to the cross section of the linear protrusion 20 (in the inverted V-shape). The groove 35 is defined by two side surfaces 35b that form the valley shape (corresponding to the side surfaces of the inner surface that defines the groove 35). The side surfaces 35b are adjacent to each other and joined to each other at the bottom of the groove 35 (a valley bottom). The groove 35 has a width that decreases toward the bottom 35a of the groove 35.

The side surfaces 35b that define the groove 35 includes rough surface sections 36 with the surface roughness greater than the surface roughness of the inner wall surface 32 (32a). The rough surface sections 36 are present for the entire areas of the side surfaces 35b of each groove 35. The surface roughness of the rough surface sections 36 is constant for the entire areas of the rough surface sections 36. In FIG. 5(a), the rough surface section 36 is indicated with dot-hatching patterns.

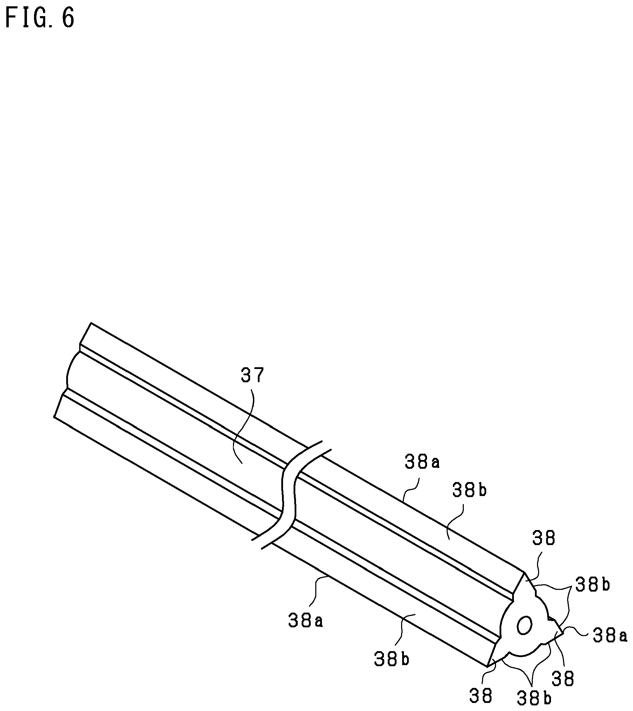

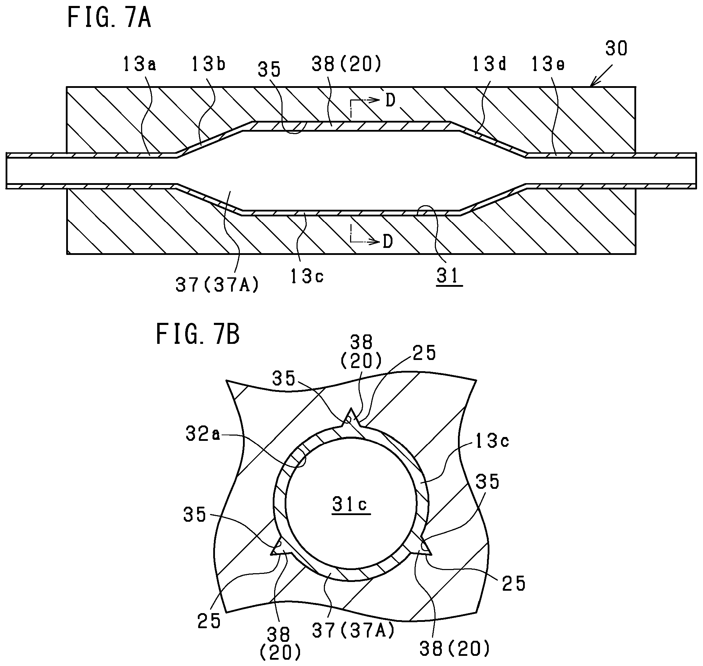

A method of producing the balloon 13 using the mold 30 will be described with reference to FIG. 6 and FIGS. 7(a) and 7(b). FIG. 6 is a perspective view illustrating a parison 37. FIG. 7(a) is a cross-sectional view illustrating the parison 37 that is expanded in the internal space of the mold 30. FIG. 7(b) is a cross-sectional view cut along line D-D in FIG. 7(a).

Prior to the production of the balloon 13, a preparing process is performed to prepare the mold 30 and the parison 37 that is a base material of the balloon 13. As illustrated in FIG. 6, the parison 37 is made of a resin material and formed in a cylindrical shape. The parison 37 may be formed by extrusion molding. The parison 37 includes multiple (specifically, three) protrusions 38 that protrude from the outer periphery of the parison 37. The protrusions 38 are to be formed into the linear protrusions 20 of the balloon 13. Each protrusion 38 has a cross section (specifically, a cross section along a direction perpendicular to the longitudinal direction of the parison 37) in an inverted V-shape, which protrudes outward in the radial direction of the parison 37. More specifically, the cross section of the protrusion 38 has a pentagonal shape, or a home base shape. The protrusion 38 extends in the longitudinal direction of the parison 37, more specifically, extends for the entire length in the longitudinal direction of the parison 37. The protrusion 38 includes two side surfaces 38b (corresponding to a surface of the protrusion 38) that form the inverted V-shape. The side surfaces 38b are adjacent to each other and joined to each other at the peak 38a of the protrusion 38.

A placing process is performed to place the parison 37 in the internal space 31 of the mold 30. In this process, the parison 37 is placed in the internal space 31 with the longitudinal direction of the parison 37 aligned with the longitudinal direction of the internal space 31. Therefore, the parison 37 is placed over the space sections 31a to 31e in the internal space 31. Then, an expanding process is performed to expand the parison 37 in the longitudinal direction.

The expanding process is performed to expand the parison 37 in the internal space 31 of the mold 30 until the outer periphery of the parison 37 is pressed against the inner wall surface 32 as illustrated in FIGS. 7(a) and 7(b). In this process, the parison 37 is expanded by introducing a pressurized fluid such as nitrogen into the inside of the parison 37 to pressurize the inside of the parison 37. Through the expanding process, an expanded parison 37A that includes the portions 13a to 13d of the balloon 13 is formed.

In the expanding process, the pressurized fluid is introduced into the inside of the parison 37 using a fluid supply device (not illustrated). The fluid supply device is capable of adjusting the pressure for pressurizing the inside of the parison 37 by adjusting an amount of the pressurized fluid introduced into the inside of the parison 37. In the expanding process, the parison 37 is heated by a heater (not illustrated) included in the mold 30 to expand the parison 37. The heater is capable of adjusting the temperature for heating the parison 37.

As illustrated in FIG. 7(b), through the expansion of the parison 37, the protrusions 38 of the parison 37 are pushed into the respective grooves 35 and the protrusions 38 are formed into the shapes of the linear protrusions 20 of the balloon 13. Through the expanding process, the linear protrusions 20 are formed on the straight tube portion 13c of the balloon 13. In the expanding process, the rough surface regions 25 are also formed in the side surfaces 20b of the linear protrusions 20 by pushing the protrusions 38 of the parison 37 into the grooves 35 and pressing the protrusions 38 against the side surfaces 35b (the rough surface sections 36) that define the grooves 35.

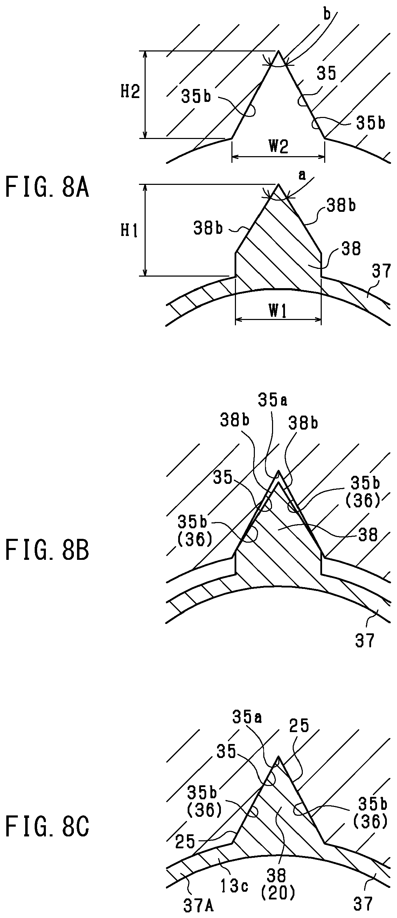

Steps of the expanding process will be described in detail with reference to FIGS. 8(a) to 8(c). FIGS. 8(a) to 8(c) are cross-sectional views illustrating how the protrusion 38 of the parison 37 is pushed into the groove 35 of the mold 30 in the expanding process.

A dimensional relationship between the protrusion 38 of the parison 37 and the groove 35 of the mold 30 will be described with reference to FIG. 8(a). As illustrated in FIG. 8(a), the width W1 (the maximum width) of the protrusion 38 is less than the width W2 (the maximum width) of the groove 35. The height H1 of the protrusion 38 is greater than the depth H2 of the groove 35. The height H1 is measured from the outer periphery of the parison 37. An angle a that is formed by the side surfaces 38b of each protrusion 38 is greater than an angle b that is formed by the side surfaces 35b of each groove 35.

A flow of the expanding process will be described. A first expanding step in the expanding process illustrated in FIG. 8(b) is performed to expand the parison 37 until the protrusions 38 of the parison 37 is inserted into the respective grooves 35. In the first expanding step, the parison 37 is pressurized at a first pressure P1, which is relatively low, to expand the parison 37. With the low pressure, the parison 37 relatively slowly expands and thus the protrusions 38 are properly inserted into the grooves 35. Because the width W1 of the protrusions 38 is less than the width W2 of the grooves 35, the protrusions 38 is easily inserted into the grooves 35. In the first expanding step, the parison 37 is heated at a first temperature T1, which is relatively low, to expand the parison 37.

In the first expanding step, the pressure inside the parison 37 (i.e., the first pressure P1) is relatively slowly increased and the heating temperature to heat the parison 37 (i.e., the first temperature T1) is relatively slowly increased. Alternatively, the pressure inside the parison 37 and the heating temperature to heat the parison 37 in the first expanding step may be constant.

A second expanding step illustrated in FIG. 8(c) is performed to further expand the parison 37 and to push the protrusions 38 into the grooves 35 until the protrusions 38 are pressed against the side surfaces 35b that define the grooves 35 (i.e., the rough surface sections 36). Through the second expanding step, the rough surface regions 25 are formed in the side surfaces 20b of the linear protrusions 20, respectively. In the second expansion step, the parison 37 is expanded by pressurizing the inside of the parison 37 at a second pressure P2 that is higher than the first pressure P1. With the second pressure P2, the protrusions 38 are squeezed into the grooves 35 and thus the protrusions 38 are pressed against the respective side surfaces 35b. Therefore, the rough surface regions 25 are properly formed in the linear protrusions 20.

In the second expanding step, the parison 37 is heated at a second temperature T2 that is higher than the first temperature T1 to expand the parison 37. In this step, the protrusions 38 of the parison 37 are softened and then pushed into the grooves 35. Therefore, the protrusions 38 can be easily pressed against the side surfaces 35b (the rough surface sections 36). That is, this step also enables proper formation of the rough surface regions 25 in the linear protrusions 20.

In the second expanding step, the internal pressure of the parison 37 (i.e., the second pressure T2) is increased more quickly in comparison to the first expanding step, and the temperature to heat the parison 37 (i.e., the second temperature T2) is increased more quickly in comparison to the first expanding step. Alternatively, the internal pressure of the parison 37 and the heating temperature to heat the parison 37 in the second expanding step may be constant.

Because the angle a that is formed by the side surfaces 38a of each protrusion 38 is greater than the angle b that is formed by the side surfaces 35b that define the corresponding groove 35, the protrusions 38 are properly deformed during insertion of the protrusions 38 into the grooves 35 and thus the protrusions 38 are properly pressed against the side surfaces 35b (i.e., the rough surface sections 36). Therefore, the rough surface regions 25 are properly formed in the linear protrusions 20, each of which is in the inverted-V shape.

Because the angle a that is formed by the side surfaces 38b of each protrusion 38 is greater than the angle b that is formed by the side surfaces 35b of each groove 35, the protrusions 38 contact the side surfaces 35b that define the grooves 35 first (see FIG. 8(b)) and then bottoms 35a of the grooves 35 (see FIG. 8(c)) during the insertion of the protrusions 38 into the grooves 35. That is, the protrusions 38 are properly pushed until the protrusions 38 reach the bottoms 35a of the grooves 35 while the protrusions 38 deform along the side surfaces 35b that define the grooves 35. Therefore, the protrusions 38 are properly pressed against the entire areas of the side surfaces 35b that define the grooves 35 (and the rough surface sections 36) and thus the rough surface areas 25 are properly formed in the linear protrusions 20.

In the expanding process, portions of the protrusions 38 of the parison 37 that are not inserted into the grooves 35 are pressed against the inner wall surfaces 32 and squashed. Therefore, the linear protrusions 20 are not formed on the portions 13a, 13b, 13d and 13e other than straight tube portion 13c.

After the expansion process, the cutting process is performed to cut off extra end portions of the expanded parison 37A. This completes the formation of the balloon 13 and the production of the balloon 13 is complete.

Then, downstream processes are performed. The downstream processes may include a joining process for joining the balloon 13 to the catheter tube 11 and a joining process for joining the catheter tube 11 to the hub 12. At the completion of the downstream processes, a series of production steps is complete.

A method of using the balloon catheter 10 will be described, which does not form part of the invention. A procedure for dilating a section of a blood vessel with a lesion using the balloon catheter 10 will be described.

A guiding catheter is inserted into a sheath introducer that is in the blood vessel and then a tip of the guiding catheter with an opening is inserted into a coronary artery inlet. The guidewire G is then inserted into the guiding catheter and the inserted guidewire G is inserted from the coronary artery inlet to a distal section via the section with the lesion.

Then, the balloon catheter 10 is inserted into the guiding catheter along the guidewire G. After the insertion, pushing force and pulling force are applied until the balloon 13 is placed in the section with the lesion. During the insertion, the balloon 13 remains deflated. When the balloon 13 is deflated, the linear protrusions 20 are covered by the wings 21 from the outer side (see FIG. 4(b)). Therefore, the rough surface regions 25 of the linear protrusions 20 are less likely to slide on a tube wall during the insertion of the balloon 13. According to the configuration, the ease in passing of the balloon 13 is less likely to decrease even through the linear protrusions 20 include the rough surface regions 25.

After the balloon 13 has reached the section with the lesion, the balloon 13 is inflated. The linear protrusions 20 are pressed against the lesion and incisions (cracks) are created in the lesion by the linear protrusions 20. The incisions may be used as a trigger to break the lesion and to dilate the section with the lesion. Because the rough surface regions 25 of the linear protrusions 20 contact the lesion, the balloon 13 is less likely to slip. According to the configuration, the section with the lesion can be properly dilated at a proper position.

After the dilation of the section with the lesion using the balloon 13 is complete, the balloon 13 is deflated. Then, the balloon catheter 10 with the deflated balloon 13 is removed from the body. This completes a series of steps.

The balloon catheter 10 may be passed through a blood vessel for treatment, for example, treatment for a coronary artery, a femoral artery, a pulmonary artery, or other types of blood vessels. The balloon catheter 10 may be used in treatment of any other types of tubes in living organism other than blood vessels such as ureters and gastrointestinal tract and body cavities.

According to the configuration of the embodiment described in detail above, the following advantageous effects can be achieved.

As described above, the rough surface regions 25 of the linear protrusions 20 contact the lesion when the balloon 13 is inflated and thus the balloon 13 is less likely to slip. Further, the surface roughness of the surface of the straight tube portion 13c of the balloon 13 is less than the surface roughness of the rough surface regions 25 of the linear protrusions 20. The surface of the straight tube portion 13c has a significantly large area in comparison to the surfaces of the linear protrusions 20. Because the surface roughness of the surface of the straight tube portion 13c is less, the sliding resistance of the balloon 13 during passing of the balloon 13 through the blood vessel can be reduced. Therefore, the slipping of the balloon 13 during the inflation of the balloon 13 can be reduced without a reduction in ease in passing of the balloon 13 through the blood vessel.

When the parison 37 is expanded and the protrusions 38 are pushed into the grooves 35 of the mold 30, the protrusions 38 are pressed against the rough surfaces 36 in the grooves 35. This forms the rough surface regions 25 in the side surfaces 20b of the linear protrusions 20. A downstream process such as scraping of the side surfaces 20b of the linear protrusions 20 to form the rough surface regions 25 is not required after the balloon 13 with the linear protrusions 20 is formed by expanding the parison 37. Therefore, the balloon 13 with the linear protrusions 20 that include the rough surface regions 25 is relatively easily produced.

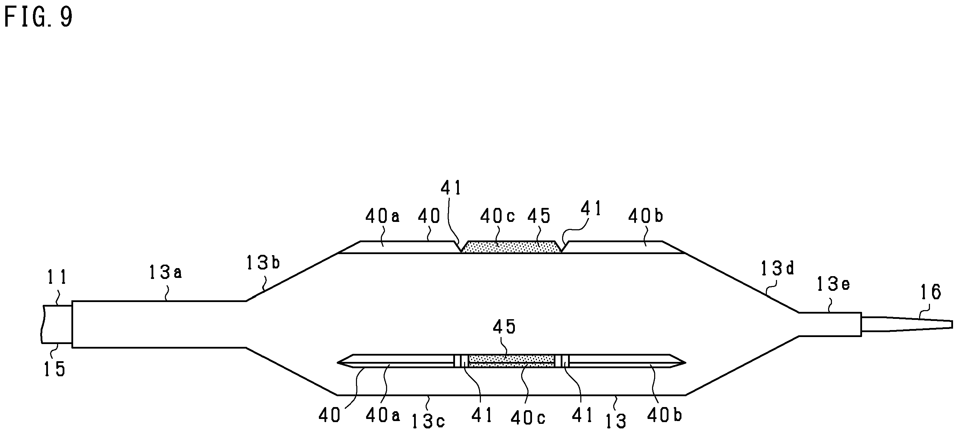

The present disclosure is not limited to the above embodiments and may be implemented as the followings, for example.
(1) In the above embodiment, the rough surface regions 25 are formed in the entire areas of the surfaces of the linear protrusions 20 (specifically, the entire areas of the side surfaces 20b). However, rough surface regions may be formed in sections of the surfaces of the linear protrusions 20. An example is illustrated in FIG. 9. The example illustrated in FIG. 9 includes linear protrusions 40 with notches 41. Multiple (specifically, two) notches 41 are formed in each liner protrusion 40. In each linear protrusion 40, the notches 41 are located to divide the linear protrusion 40 into approximately equal sections (specifically, into thirds).

The linear protrusions 40 include multiple sections 40a to 40c that are separated by the notches 41. The sections 40a to 40c include base-end sections 40a, tip-end sections 40b, and intermediate sections 40c. The base-end sections 40a are located on the base-end side. The tip-end sections 40b are located on the tip-end side. The intermediate sections 40c are located between the base-end sections 40a and the tip-end sections 40b. Rough surface regions 45 are formed in surfaces of the intermediate sections 40c. The surface roughness of the rough surface regions 45 is greater than the surface roughness of the surface of the straight tube portion 13c. The rough surface regions 45 are not formed in the surfaces of the base-end sections 40a and the tip-end sections 40b. That is, only the intermediate sections 40c include the rough surface regions 45 in this embodiment.

According to the configuration, even if the tip-end sections 40b or the base-end sections 40a of the linear protrusions 40 stick out of the wings 21 and slide on a tube wall during insertion of the deflated balloon 13 into a blood vessel, the sliding resistance is less likely to increase. Therefore, the ease in passing of the balloon 13 through the blood vessel is less likely to decrease.

(2) In the configuration of the above embodiment, the rough surface regions 25 may be configured such that the surface roughness decreases toward the ends of the linear protrusions 20 with respect to the longitudinal direction. For example, the surface roughness of the rough surface regions 25 may be at the maximum at the middle of the linear protrusions 20 with respect to the longitudinal direction. The surface roughness of each rough surface region 25 may decrease from the middle toward one of the ends of the linear protrusions 20 and from the middle toward the other end of the linear protrusions 20 with respect to the longitudinal direction. According to such a configuration, effects similar to the effects described in (1) can be achieved.

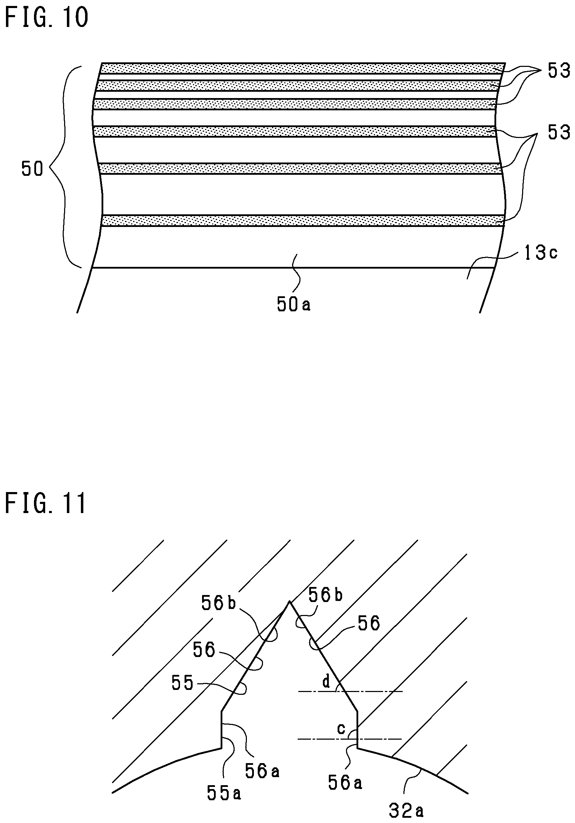

(3) Multiple rough surface regions may be formed in each surface of the linear protrusions. An example is illustrated in FIG. 10. FIG. 10 is a magnified side view of a liner protrusion 50 that includes multiple rough surface regions 53. The example illustrated in FIG. 10 includes the rough surface regions 53 in each side surface 50a of the linear protrusion 50. Each rough surface region 53 extends in the longitudinal direction of the linear protrusion 50, specifically, extends for an entire length of the linear protrusion 50 in the longitudinal direction. The rough surface regions 53 are arranged at predefined intervals in the side surface 50a of the linear protrusion 50. The rough surface regions 53 are arranged such that intervals between adjacent ones of the rough surface regions 53 decrease toward the peak of the linear protrusion 50. That is, the rough surface regions 53 are arranged such that the density of the rough surface regions 53 increases toward the peak of the linear protrusion 50.

When the balloon 13 is inflated to press the linear protrusion 50 against the lesion, the peak of the linear protrusion 50 primarily contacts the lesion. In the configuration described above, the rough surface regions 53 are arranged such that the density of the rough surface regions 53 increases toward the peak of the linear protrusion 50. Therefore, the balloon 13 is less likely to slip during the inflation of the balloon 13.

The rough surface regions 53 are arranged such that the density of the rough surface regions 53 decreases toward the base of the linear protrusion 50 (i.e., opposite from the peak). Therefore, the sliding resistance of the balloon 13 during the insertion of the balloon 13 into a blood vessel can be reduced in comparison to a configuration in which the rough surface regions 53 are present on each side surface 50a of the linear protrusion 50 with high density for an entire area of the side surface 50a. According to the configuration, the slipping of the balloon 13 is properly reduced without a decrease in ease in passing of the balloon 13.

(4) In the configuration of the above embodiment, the rough surface region 25 may be configured such that the surface roughness increases toward the peak of the linear protrusion 20. According to the configuration, the slipping of the balloon 13 during the inflation of the balloon 13 is properly reduced. In this configuration, the surface roughness of the rough surface region 25 decreases toward the base of the linear protrusion 20. Therefore, the sliding resistance of the balloon 13 during the insertion of the balloon 13 into a blood vessel can be reduced in comparison to a configuration in which the entire rough surface region 25 has greater surface roughness. According to the configuration, similar to the configuration described in (3), the slipping of the balloon 13 is further properly reduced without a decrease in ease in passing of the balloon 13.

(5) The relationship between the dimension of each groove 35 of the mold 30 and the dimension of the corresponding protrusion 38 of the parison 37 is not limited to the relationship in the above embodiment. For example, the height H1 of the protrusion 38 may be less than the depth H2 of the groove 35 or equal to the depth H2 of the groove 35. According to the configuration, the entire protrusion 38 can be properly inserted into the groove 35. The angle a that is formed by the side surfaces 38b of the protrusion 38 may be less than the angle b that is formed by the side surfaces 35b that define the groove 35. According to the configuration, the protrusion 38 of the parison 37 is easily inserted into the groove 35.

(6) The mold 30 may include a groove that has a cross section illustrated in FIG. 11. An example illustrated in FIG. 11 includes a groove 55 that has a cross section in a V-shape similar to that of the embodiment described above. That is, the groove 55 is defined by two side surfaces 56 that form the V-shape. The angle of each side surface 56 relative to the width direction of the groove 55 varies. Specifically, the angle varies between a side surface section 56a of the side surface 56 at an opening section 55a of the groove 55 and a side surface section 56b of the side surface 56 located on a bottom side of the groove 55 than the opening section 55a. More specifically, an angle c of the side surface section 56a relative to the width direction of the groove 55 is greater than an angle d of the side surface section 56b relative to the width direction of the groove 55. For example, the angle c may be 90°.

According to the configuration, the protrusion 38 of the parison 37 inserted into the groove 55 in the expanding process is less likely to slide on the side surfaces 56 that define the groove 55. Therefore, problems such as improper formation of the rough surface regions 25 in the side surfaces 20b of the linear protrusion 20 due to the sliding of the protrusion 38 in the groove 55 are less likely to occur.

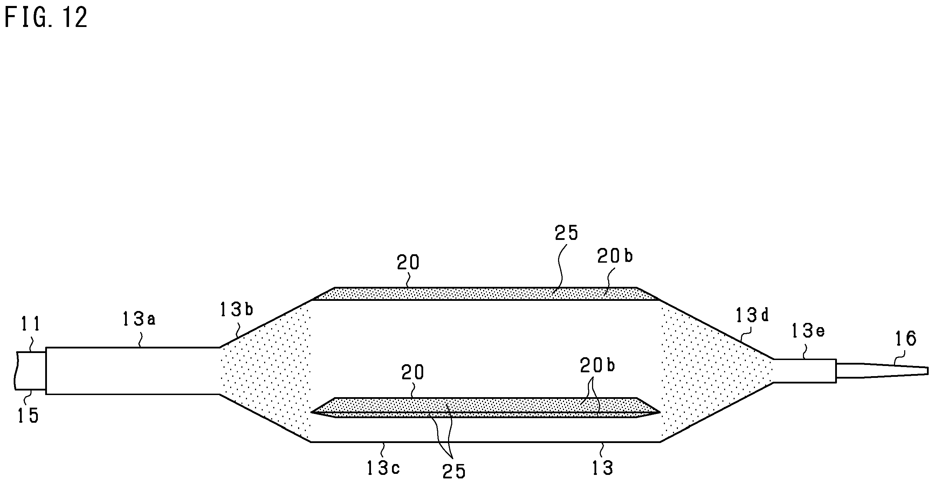

(7) During the dilation of the section with the lesion by the tapered portions 13b, 13d of the balloon 13, the tapered portions 13b, 13d contact the lesion at an angle and thus the lesion may push back the tapered portions 13b, 13d, which may causes slipping of the balloon 13 in the axial direction. To reduce such a problem, the surfaces of the tapered portions 13b, 13d of the balloon 13 may be configured such that the surface roughness is greater than the surface roughness of the surface of the straight tube portion 13c as illustrated in FIG. 12. According to the configuration, the surfaces of the tapered portions 13b, 13d contact the lesion and thus the tapered portions 13b, 13d are less likely to slip in the axial direction. Further, the balloon 13 is less likely to slip in the axial direction. In FIG. 12, the rough surfaces 13b, 13d are indicated with dot-hatching patterns.

In the example illustrated in FIG. 12, the surface roughness of the surfaces of the tapered portions 13b, 13d is less than the surface roughness of the surfaces of the rough surface regions 25 of the linear protrusions 20. According to the configuration, the effects described earlier can be achieved without a reduction in ease in passing of the balloon 13 through a blood vessel. Alternatively, the surface roughness of only either one of the tapered portions 13b, 13d may be increased.

(8) In the above embodiment, the linear protrusions 20 (first linear protrusions) protrude from the surface of the straight tube portion 13c of the balloon 13. The configuration may further include linear protrusions (second linear protrusions) that protrude from the surface of the tip-end tapered portion 13d. In such a configuration, the surfaces of the second linear protrusions may include rough surface regions with surface roughness greater than the surface roughness of the surface of the straight tube portion 13c. According to the configuration, the balloon 13 is further less likely to slip during the inflation of the balloon 13.

The configuration may also include linear protrusions (third linear protrusions) that protrude from the surface of the base-end tapered portion 13b and include surfaces that include rough surface regions with surface roughness greater than the surface roughness of the surface of the straight tube portion 13c.

(9) In the above embodiment, the rough surface regions 25 are formed in the side surfaces 20b of the linear protrusions 20 in the expanding process to expand the parison 37. However, the method of forming the rough surface regions 25 is not limited to such a method. For example, the balloon 13 with the linear protrusions 20 may be formed and then the side surfaces 20b of the linear protrusions 20 may be scraped to form the rough surface regions 25.

The present disclosure has been described with reference to the embodiments. However, the present disclosure is not limited to the embodiments or the structures. Further, various combinations, configurations, and other combinations and configurations including single elements, more or less, may be within the technical scope of the present disclosure.

### Reference Sings List

10: Balloon catheter, 13: Balloon, 13c: Straight tube portion, 20: Linear protrusion, 25: Rough surface region, 30: Mold, 31: Internal space, 35: Groove, 36: Rough surface, 37: Parison, 38: Protrusion

## Claims

1. A balloon catheter (10) comprising:
a balloon (13) that is inflatable and deflatable and includes a straight tube portion (13c) in a cylindrical shape with a diameter to be maximum when the balloon (13) is inflated; and
a linear protrusion (20) that protrudes from a surface of the straight tube portion (13c) and extends along the surface of the straight tube portion (13c), wherein the linear protrusion (20) has a surface that includes at least one rough surface region (25) with surface roughness greater than surface roughness of the surface of the straight tube portion (13c), **characterized in that**
the surface roughness of the at least one rough surface region (25) increases toward a peak of the linear protrusion (20).

2. The balloon catheter (10) according to claim 1, wherein
the balloon (13) includes a wing (21) that is formed when the balloon is deflated and folded along an outer periphery of the balloon,
the linear protrusion (20) extends in an axial direction of the balloon (13), and
the linear protrusion (20) is covered by the wing (21) from an outer side when the balloon is deflated.

3. A method of producing the balloon catheter (10) according to claim 1, wherein
a parison (37) in a tubular shape is a base material of the balloon (13) and includes a protrusion (38) on an outer periphery of the parison (37) and to be formed into the linear protrusion (20),
a mold (30) that is used for producing the balloon (13) includes an internal space (31) for forming the balloon (13),
the internal space (31) includes a space section (31c) for forming the straight tube portion (13c),
the mold (30) includes an inner wall surface (32) that defines the internal space (31) and includes a wall surface section (32a) that defines the space section (31c),
the mold (30) includes a groove (35, 55) in the wall surface section (32a) for forming the linear protrusion (20), and
the mold (30) includes an inner surface that defines the groove (35, 55) and includes a rough surface section (36) with surface roughness greater than surface roughness of the wall surface section (32a),
the method comprising an expanding process of expanding the parison (37) in the internal space (31) until the outer periphery of the parison (37) is pressed against the inner wall surface (32) and the protrusion (38) is pushed into the groove (35, 55) to form the linear protrusion (20), wherein the at least one rough surface region (25) is formed in the surface of the linear protrusion (20) by pushing of the protrusion (38) into the groove (35, 55) and pressing of the protrusion (38) against the rough surface section (36) in the expanding process.

4. The method according to claim 3, wherein the expanding process comprises:
a first expanding step of pressurizing an inside of the parison (37) at a first pressure to expand the parison until the protrusion (38) is inserted into the groove (35, 55); and
a second expanding step of pressurizing the inside of the parison (37) at a second pressure that is higher than the first pressure to push the protrusion (38) into the groove (35, 55) until the protrusion (38) is pressed against the rough surface section (36).

5. The method according to claim 4, wherein
the first expanding step includes heating the parison (37) at a first temperature (T1), and
the second expanding step includes heating the parison (37) at a second temperature (T2) that is higher than the first temperature (T1).

6. The method according to any one of claims 3 to 5, wherein the protrusion (38) has a height (H1) greater than a depth (H2) of the groove (35, 55).

7. The method according to any one of claims 3 to 5, wherein
the groove has a cross section in a V-shape,
the protrusion (38) has a cross section in an inverted V-shape,
the inner surface that defines the groove includes two side surfaces (35b) that form the V-shape,
the protrusion (38) includes a surface that includes two side surfaces (38b) that form the inverted V-shape, and
the side surfaces (38b) of the protrusion (38) form an angle (a) greater than an angle (b) formed by the side surfaces (35b) that define the groove (35, 55).

8. The method according to claim 7, wherein
an angle of each of the side surfaces relative to a width direction of the groove (55) is greater at an opening section (55a) of the groove (55) in comparison to a bottom section of the groove.

9. The method according to any one of claims 3 to 5, wherein
the groove (35) has a width that decreases toward a bottom (35a) of the groove,
the inner surface that defines the groove (35) includes two side surfaces (35b) adjacent to each other and joined to each other at the bottom (35a) of the groove (35), and
the expanding process includes pushing the protrusion (38) into the groove (35) until the protrusion (38) contacts the side surfaces (35b) that define the groove and then the bottom (35a) of the groove.

## Patentansprüche

1. Ballonkatheter (10), umfassend:
einen Ballon (13), der aufpumpbar und entleerbar ist und einen geraden Rohrabschnitt (13c) in zylindrischer Form aufweist, dessen Durchmesser im aufgepumpten Zustand des Ballons (13) sein Maximum erreicht; und
einen linearen Vorsprung (20), der von einer Oberfläche des geraden Rohrabschnitts (13c) vorsteht und sich entlang der Oberfläche des geraden Rohrabschnitts (13c) erstreckt, wobei der lineare Vorsprung (20) eine Oberfläche aufweist, die zumindest einen rauen Oberflächenbereich (25) mit einer Oberflächenrauheit umfasst, die größer ist als die Oberflächenrauheit der Oberfläche des geraden Rohrabschnitts (13c), **dadurch gekennzeichnet, dass**
die Oberflächenrauheit des zumindest einen rauen Oberflächenbereichs (25) in Richtung einer Spitze des linearen Vorsprungs (20) zunimmt.

2. Ballonkatheter (10) gemäß Anspruch 1, wobei
der Ballon (13) einen Flügel (21) umfasst, der gebildet wird, wenn der Ballon entleert und der entlang eines Außenumfangs des Ballons gefaltet ist,
sich der lineare Vorsprung (20) in einer axialen Richtung des Ballons (13) erstreckt und
der lineare Vorsprung (20) von einer Außenseite her von dem Flügel (21) bedeckt ist, wenn der Ballon entleert ist.

3. Verfahren zur Herstellung des Ballonkatheters (10) gemäß Anspruch 1, wobei
ein rohrförmiger Vorformling (37) ein Ausgangsmaterial des Ballons (13) ist und an einem Außenumfang des Vorformlings (37) einen Vorsprung (38) aufweist und zu dem linearen Vorsprung (20) geformt werden soll,
eine Form (30), die zur Herstellung des Ballons (13) verwendet wird, einen Innenraum (31) zum Formen des Ballons (13) aufweist,
der Innenraum (31) einen Raumabschnitt (31c) zum Formen des geraden Rohrabschnitts (13c) aufweist,
die Form (30) eine Innenwandfläche (32) aufweist, die den Innenraum (31) definiert und einen Wandflächenabschnitt (32a) umfasst, der den Raumabschnitt (31c) definiert,
die Form (30) in dem Wandflächenabschnitt (32a) eine Nut (35, 55) zum Formen des linearen Vorsprungs (20) aufweist, und
die Form (30) eine Innenfläche aufweist, die die Nut (35, 55) definiert und einen rauen Oberflächenabschnitt (36) mit einer Oberflächenrauheit aufweist, die größer ist als die Oberflächenrauheit des Wandflächenabschnitts (32a),
wobei das Verfahren einen Aufweitungsvorgang umfasst, bei dem der Vorformling (37) im Innenraum (31) aufgeweitet wird, bis der Außenumfang des Vorformlings (37) gegen die Innenwandfläche (32) gedrückt wird und der Vorsprung (38) in die Nut (35, 55) gedrückt wird, um den linearen Vorsprung (20) zu bilden, wobei der zumindest eine raue Oberflächenbereich (25) in der Oberfläche des linearen Vorsprungs (20) durch das Drücken des Vorsprungs (38) in die Nut (35, 55) und das Drücken des Vorsprungs (38) gegen den rauen Oberflächenabschnitt (36) im Aufweitungsvorgang gebildet wird.

4. Verfahren gemäß Anspruch 3, wobei der Aufweitungsvorgang umfasst:
einen ersten Aufweitungsschritt, bei dem ein Inneres des Vorformlings (37) mit einem ersten Druck beaufschlagt wird, um den Vorformling aufzuweiten, bis der Vorsprung (38) in die Nut (35, 55) eingeführt ist; und
einen zweiten Aufweitungsschritt, bei dem das Innere des Vorformlings (37) mit einem zweiten Druck beaufschlagt wird, der höher ist als der erste Druck, um den Vorsprung (38) in die Nut (35, 55) zu drücken, bis der Vorsprung (38) gegen den rauen Oberflächenabschnitt (36) gedrückt wird.

5. Verfahren gemäß Anspruch 4, wobei
der erste Aufweitungsschritt das Wärmen des Vorformlings (37) auf eine erste Temperatur (T1) umfasst, und
der zweite Aufweitungsschritt das Wärmen des Vorformlings (37) auf eine zweite Temperatur (T2) umfasst, die höher ist als die erste Temperatur (T1).

6. Verfahren gemäß einem der Ansprüche 3 bis 5, wobei der Vorsprung (38) eine Höhe (H1) aufweist, die größer ist als eine Tiefe (H2) der Nut (35, 55).

7. Verfahren gemäß einem der Ansprüche 3 bis 5, wobei
die Nut einen V-förmigen Querschnitt aufweist,
der Vorsprung (38) einen umgekehrt V-förmigen Querschnitt aufweist,
die Innenfläche, die die Nut definiert, zwei Seitenflächen (35b) umfasst, die die V-Form bilden,
der Vorsprung (38) eine Oberfläche aufweist, die zwei Seitenflächen (38b) umfasst, die die umgekehrte V-Form bilden, und
die Seitenflächen (38b) des Vorsprungs (38) einen Winkel (a) bilden, der größer ist als ein Winkel (b), der von den Seitenflächen (35b) gebildet wird, die die Nut (35, 55) definieren.

8. Verfahren gemäß Anspruch 7, wobei
ein Winkel jeder der Seitenflächen relativ zu einer Breitenrichtung der Nut (55) an einem Öffnungsabschnitt (55a) der Nut (55) größer ist als an einem Bodenabschnitt der Nut.

9. Verfahren gemäß einem der Ansprüche 3 bis 5, wobei
die Nut (35) eine Breite aufweist, die zum Boden (35a) der Nut hin abnimmt,
die die Nut (35) definierende Innenfläche zwei nebeneinanderliegende Seitenflächen (35b) umfasst, die am Boden (35a) der Nut (35) miteinander verbunden sind, und
der Aufweitungsvorgang das Drücken des Vorsprungs (38) in die Nut (35) umfasst, bis der Vorsprung (38) die die Nut definierenden Seitenflächen (35b) und anschließend den Boden (35a) der Nut berührt.

## Revendications

1. Cathéter à ballonnet (10) comprenant :
un ballonnet (13) qui est gonflable et dégonflable et comprend une partie de tube droite (13c) de forme cylindrique avec un diamètre devant être maximal lorsque le ballonnet (13) est gonflé ; et
une saillie linéaire (20) qui fait saillie d'une surface de la partie de tube droite (13c) et s'étend le long de la surface de la partie de tube droite (13c), dans lequel la saillie linéaire (20) a une surface qui comprend au moins une région de surface rugueuse (25) avec une rugosité de surface supérieure à la rugosité de surface de la surface de la partie de tube droite (13c), **caractérisé en ce que**
la rugosité de surface de l'au moins une région de surface rugueuse (25) augmente vers un pic de la saillie linéaire (20).

2. Cathéter à ballonnet (10) selon la revendication 1, dans lequel
le ballonnet (13) comprend une aile (21) qui est formée lorsque le ballonnet est dégonflé et pliée le long d'une périphérie externe du ballonnet,
la saillie linéaire (20) s'étend dans une direction axiale du ballonnet (13), et
la saillie linéaire (20) est recouverte par l'aile (21) depuis un côté externe lorsque le ballonnet est dégonflé.

3. Procédé de production du cathéter à ballonnet (10) selon la revendication 1, dans lequel
une paraison (37) de forme tubulaire est un matériau de base du ballonnet (13) et comprend une saillie (38) sur une périphérie externe de la paraison (37) et devant être formée en la saillie linéaire (20),
un moule (30) qui est utilisé pour produire le ballonnet (13) comprend un espace interne (31) pour former le ballonnet (13),
l'espace interne (31) comprend une section d'espace (31c) pour former la partie de tube droite (13c),
le moule (30) comprend une surface de paroi interne (32) qui définit l'espace interne (31) et comprend une section de surface de paroi (32a) qui définit la section d'espace (31c),
le moule (30) comprend une rainure (35, 55) dans la section de surface de paroi (32a) pour former la saillie linéaire (20), et
le moule (30) comprend une surface interne qui définit la rainure (35, 55) et comprend une section de surface rugueuse (36) avec une rugosité de surface supérieure à la rugosité de surface de la section de surface de paroi (32a),
le procédé comprenant un processus d'expansion consistant à expanser la paraison (37) dans l'espace interne (31) jusqu'à ce que la périphérie externe de la paraison (37) soit pressée contre la surface de paroi interne (32) et la saillie (38) soit poussée dans la rainure (35, 55) pour former la saillie linéaire (20), dans lequel l'au moins une région de surface rugueuse (25) est formée dans la surface de la saillie linéaire (20) en poussant la saillie (38) dans la rainure (35, 55) et en pressant la saillie (38) contre la section de surface rugueuse (36) dans le processus d'expansion.

4. Procédé selon la revendication 3, dans lequel le processus d'expansion comprend :
une première étape d'expansion consistant à mettre sous pression un intérieur de la paraison (37) à une première pression pour expanser la paraison jusqu'à ce que la saillie (38) soit insérée dans la rainure (35, 55) ; et
une seconde étape d'expansion consistant à mettre sous pression l'intérieur de la paraison (37) à une seconde pression qui est supérieure à la première pression pour pousser la saillie (38) dans la rainure (35, 55) jusqu'à ce que la saillie (38) soit pressée contre la section de surface rugueuse (36).

5. Procédé selon la revendication 4, dans lequel
la première étape d'expansion comprend le chauffage de la paraison (37) à une première température (T1), et
la seconde étape d'expansion comprend le chauffage de la paraison (37) à une seconde température (T2) qui est supérieure à la première température (T1).

6. Procédé selon l'une quelconque des revendications 3 à 5, dans lequel la saillie (38) a une hauteur (H1) supérieure à une profondeur (H2) de la rainure (35, 55).

7. Procédé selon l'une quelconque des revendications 3 à 5, dans lequel
la rainure a une section transversale en forme de V,
la saillie (38) a une section transversale en forme de V inversé,
la surface interne qui définit la rainure comprend deux surfaces latérales (35b) qui forment la forme de V,
la saillie (38) comprend une surface qui comprend deux surfaces latérales (38b) qui forment la forme de V inversé, et
les surfaces latérales (38b) de la saillie (38) forment un angle (a) supérieur à un angle (b) formé par les surfaces latérales (35b) qui définissent la rainure (35, 55).

8. Procédé selon la revendication 7, dans lequel
un angle de chacune des surfaces latérales par rapport à une direction de largeur de la rainure (55) est supérieur au niveau d'une section d'ouverture (55a) de la rainure (55) par rapport à une section de fond de la rainure.

9. Procédé selon l'une quelconque des revendications 3 à 5, dans lequel
la rainure (35) a une largeur qui diminue vers un fond (35a) de la rainure,
la surface interne qui définit la rainure (35) comprend deux surfaces latérales (35b) adjacentes l'une à l'autre et jointes l'une à l'autre au niveau du fond (35a) de la rainure (35), et
le processus d'expansion comprend la poussée de la saillie (38) dans la rainure (35) jusqu'à ce que la saillie (38) vienne en contact avec les surfaces latérales (35b) qui définissent la rainure puis le fond (35a) de la rainure.
